# EUROPEAN PATENT SPECIFICATION

(11) **EP 4 504 121 B1**
(45) Date of publication and mention of the grant of the patent: **05.08.2026**
(21) Application number: 23718810.7
(22) Date of filing: 05.04.2023
(51) Int. Cl.: A61J 1/10, A61J 1/20, B65D 81/32, A61B 1/12

(54) **CLEANING APPARATUS FOR CLEANING INTERNAL CHANNELS OF AN ITEM OF EQUIPMENT**
REINIGUNGSVORRICHTUNG ZUR REINIGUNG VON INNENKANÄLEN EINES AUSRÜSTUNGSTEILS
APPAREIL DE NETTOYAGE POUR NETTOYER DES CANAUX INTERNES D'UN ARTICLE D'ÉQUIPEMENT

(30) Priority: 06.04.2022 GB 202205049
(43) Date of publication of application: 12.02.2025
(73) Proprietor: Meditech Endoscopy Limited, Tapton, Chesterfield S41 0TZ (GB)
(72) Inventor: RAMSEY, Peter, Tapton, Chesterfield S41 0TZ (GB)
(74) Representative: Dummett Copp LLP
(86) International application number: PCT/GB2023/050913
(87) International publication number: WO 2023/194731

(56) References cited:
- WO-A1-00/56203
- US-A1- 2005 087 458
- US-A1- 2013 126 370

## Description

### FIELD OF THE INVENTION

The present invention relates to a cleaning apparatus for flushing and cleaning the internal channels of an item of equipment such as an endoscope or similar instrument. This invention also relates to a method of cleaning internal channels of a piece of equipment such as an air/water channel of an endoscope.

### BACKGROUND TO THE INVENTION

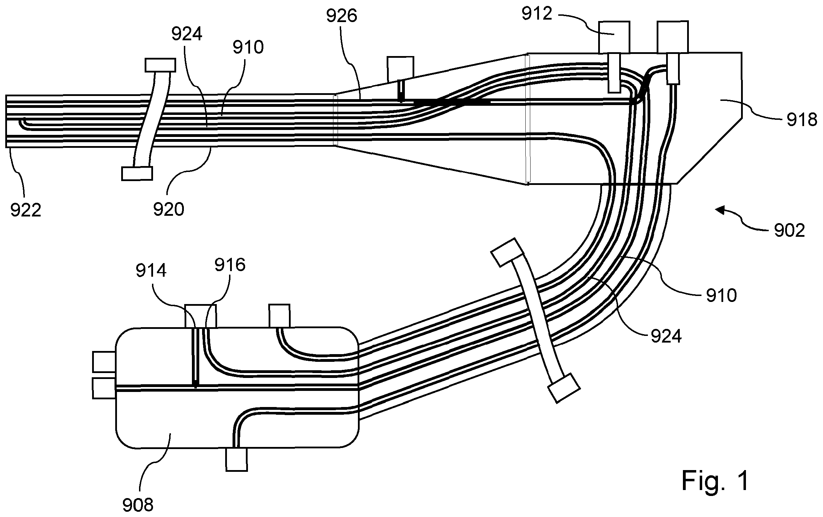

Figure 1 illustrates an air/water system of an endoscope 902. A first component of such an endoscope air/water system is an air pump 904 in a light source 906. The air flows into the endoscope 902 through a light guide connector 908 of the endoscope 902. The air travels through a very small diameter channel 910 in the endoscope 902 and flows out of an air/water valve 912. The air/water system is controlled from a control section 918 of the endoscope 902. During use of the endoscope 902, in order to insufflate an organ to be examined, the air/water valve 912 is gently covered thereby preventing air exiting through the air/water valve 912 and instead diverting air down the insertion tube 920 and out of a distal tip 922 of the endoscope 902.

Another component of the air/water system is a water bottle. The water bottle is attached to air and water ports 914, 916 of the endoscope 902. During use, the air/water valve 912 may be depressed so that air entering the endoscope 902 is diverted through the air port 914 of the endoscope 902 and into the water bottle causing the bottle to be pressurized. This pressure then forces water from the bottle, through the water port 916, and into another small diameter channel 924 in the endoscope. The water travels along the channel 924 and may be used to flush or clear the view at the distal tip 922 of the endoscope 902.

The air/water channel is therefore actually formed by the two separate small diameter channels 910, 924 that combine into a single channel only proximate the distal tip 922 of the endoscope 902. Furthermore, a small metal nozzle is generally disposed at the tip 922 of the endoscope 902 to deflect air or water from the air/water channel 910, 924 across the image lens of the endoscope 902. The presence of this metal nozzle means that it is not possible to brush the air/water channel 910, 924. This, together with the small diameter of the air/water channel 910, 924, as well as other factors, makes it difficult to clean and decontaminate the air/water channel 910, 924.

The air/water nozzle is the area of greatest failure in the channel system of endoscopes as the air/water nozzle is the point of smallest internal diameter of the air/water channel system, which means that it can easily become blocked or obstructed by debris. Furthermore, during an endoscopic procedure, air infused into the organ being examined can force debris to back up into the nozzle and into the air/water channels 910, 924. This debris can travel some distance back into the channels.

The endoscope also includes a suction/biopsy channel 926 that extends to the tip 922 of the endoscope 902. It will be appreciated that it is very important to thoroughly clean and sterilise this suction/biopsy channel 926 to prevent cross-contamination.

Accordingly, after use of an endoscope, the endoscope must be cleaned and sterilised. Cleaning refers to the removal of visible debris, which may include both inorganic and organic substances. Cleaning usually involves a mechanical process, together with water and detergent. Following cleaning, sterilisation of the endoscope destroys microorganisms present on or in the endoscope. Sterilisation processes usually involve steam or a chemical sterilant. Following sterilisation, the endoscope undergoes high-level disinfection.

It is currently recommended that a preliminary cleaning routine should be undertaken immediately after use and before the endoscope is detached from the light source/video processor. Current pre-clean practice is to flush air and water through both the air/water channel and the suction/biopsy channel. It has been found, however, that routine pre-cleaning procedures for endoscopes do not remove biofilm reliably from endoscope channels. This leads to failures in the subsequent decontamination processes.

It is also recommended that the air and water channels, suction/biopsy channel, and any auxiliary channel, are also irrigated with detergent, not only to check for blockages but also to expel any blood, mucus and other debris. For example, WO 00/56203 A1 discloses a container pre-packaged with a measured dose of a concentrated cleanser. In use, the container is opened so that the internal volume of the container may be filled with a suitable volume of water to mix with the cleanser to form a cleaning solution. A fill line on the container may be used to indicate the required volume of water.

It is an object of the present invention to provide a cleaning apparatus that permits improved cleaning of internal channels of an endoscope, in particular with a detergent solution, and that overcomes at least one disadvantage of prior art endoscope channel flushing or cleaning systems whether referred to herein or otherwise.

### SUMMARY OF THE INVENTION

The invention provides a cleaning apparatus for cleaning internal channels of an item of equipment as defined in claim 1.

Preferably the internal volume of the reservoir is no more than 10% of the internal volume of the main body portion. In preferred embodiments the internal volume of the reservoir holds 5ml of liquid and the internal volume of the main body portion holds 500ml of liquid.

In some embodiments the reservoir comprises a pouch that is suspended from a part of the neck portion of the bag.

In some embodiments the neck portion of the bag is closed at a top furthest from the main body portion by a heat welded seal.

According to the present invention the neck portion of the bag includes a frangible seal that forms a boundary of the reservoir. The frangible seal preferably includes a weld line having an arc-shaped portion forming a concavity for receiving the second liquid. In preferred embodiments the neck portion of the bag is closed at a top furthest from the main body portion by a heat welded seal, such that the reservoir is defined between the frangible seal and the heat welded seal.

The neck portion of the bag comprises a retainer arranged to engage with said item of equipment to hold a part of said item of equipment in said mixed volume of detergent and sterile or distilled water.

According to the present invention the cleaning apparatus comprises a volume of the first liquid in the internal volume of the main body portion and a volume of the second liquid in the internal volume of the reservoir. The first liquid is sterile water or distilled water and the second liquid is a detergent.

The present invention further provides a method of manufacturing a cleaning apparatus as defined in claim 7.

Preferably the top of the neck portion is sealed by heat welding.

In preferred embodiments the frangible seal comprises a weld line that extends in a direction generally across the neck portion with a central region of the weld line having a semi-circular shape that extends generally in a direction towards the main body portion and defines a recess of the frangible seal, and the method comprises filling the recess with the second liquid.

According to the present invention the first liquid is sterile water or distilled water and the second liquid is a concentrated detergent.

The present invention also provides a method of using a cleaning apparatus as defined in claim 10.

In preferred embodiments the neck portion of the bag is closed at a top furthest from the main body portion by a heat welded seal, and the method further comprises breaking the heat welded seal or separating the heat welded seal from the bag.

In preferred embodiments the piece of equipment is an endoscope and the method comprises inserting a tip of the endoscope into the liquid in the main body portion of the bag.

The neck portion of the bag comprises a retainer and the method comprises engaging the retainer with the item of equipment to retain a part of the item of equipment in the first liquid.

Preferred and/or optional features of each embodiment described above may also be used, alone or in appropriate combination, in the other aspects and embodiments also.

### BRIEF DESCRIPTION OF THE DRAWINGS

The invention will now be further described by way of example only and with reference to the accompanying drawings, in which like reference signs are used for like features, and in which:
Figure 1 illustrates an air/water system of an endoscope;
Figures 2 to 4 illustrate a cleaning apparatus in an initial state before use according to an embodiment of the present invention, the cleaning apparatus comprising a bag for holding a volume of fluid;
Figures 5 to 7 illustrate the cleaning apparatus of Figures 2 to 4 in a second state in use, with a tip of an endoscope disposed within the internal volume of the bag;
Figures 8 to 13 show the steps in a method of manufacturing a cleaning apparatus according to a further embodiment of the present invention, the cleaning apparatus comprising a bag for holding a volume of fluid;
Figures 14 and 15 show the cleaning apparatus of Figures 8 to 13 in an initial state before use; and
Figures 16 to 19 show the steps in a method of using the cleaning apparatus of Figures 8 to 13.

### DESCRIPTION OF THE PREFERRED EMBODIMENTS

The present invention provides a cleaning apparatus that comprises a bag or container for holding a volume of a liquid, which is a detergent solution. In use, a tip or part of a piece of equipment, such as a tip of an endoscope, may be inserted into the internal volume of the container and is preferably retained in a position such that the tip or part of the piece of equipment is held in the volume of liquid. The liquid may be used to clean external surfaces of the piece of equipment. Additionally or alternatively, the liquid may be drawn up through an internal channel of the equipment to flush or clean the channel of the equipment.

When used to clean medical equipment, such as an endoscope or similar, the bag may contain a neutral pH enzymatic detergent solution. The detergent is designed to break down proteinaceous materials and prevent the formation of biofilm, for example in the air/water channel of the endoscope.

For most uses, the detergent solution in the bag is preferably of a relatively low concentration. It has been found, however, that pre-mixed low concentration detergent degrades over time. Tests show that bacterial growth occurs in low concentration detergent. Accordingly, if the cleaning apparatus is stored for a long time before use, the detergent solution in the cleaning apparatus may have degraded to a point at which the cleaning apparatus is no longer fit for purpose.

Accordingly, it is desirable if the low concentration detergent solution is used soon after it is formed. One option is to separate, within the container or bag, a volume of sterile water and a volume of concentrated detergent. These two volumes could then be mixed to create the required low concentration detergent solution shortly before using the cleaning apparatus.

The cleaning apparatus is therefore advantageously provided with a reservoir within the internal volume of the bag. The bag preferably includes a main body portion that provides an internal volume for holding a first liquid. The reservoir preferably has an internal volume for holding a second liquid. In preferred embodiments the first liquid is sterile water and the second liquid is a concentrated detergent.

In an initial state the reservoir holds the second liquid separated from the first liquid. Before use of the cleaning apparatus, the reservoir is preferably ruptured, broken or opened to permit the second liquid to mix with the first liquid. Accordingly, in embodiments in which the first liquid is sterile water and the second liquid is a concentrated detergent, these may be mixed shortly before use to form the lower concentration detergent solution within the internal volume of the bag.

According to the present invention the bag comprises an upper neck portion providing access to the main body portion of the bag and the reservoir is engaged with the neck portion of the bag. In preferred embodiments the reservoir is configured such that, in use, when the neck portion is opened to access the internal volume of the main body portion, the reservoir is concurrently opened such that the second liquid is released from the reservoir to mix with the first liquid.

The internal volume of the reservoir is preferably significantly smaller than the internal volume of the main body portion. The internal volume of the reservoir may be smaller than 20% of the internal volume of the bag. The internal volume of the reservoir may be smaller than 10% of the internal volume of the bag. The internal volume of the reservoir may hold 5ml of liquid and the internal volume of the bag may hold 500ml of liquid.

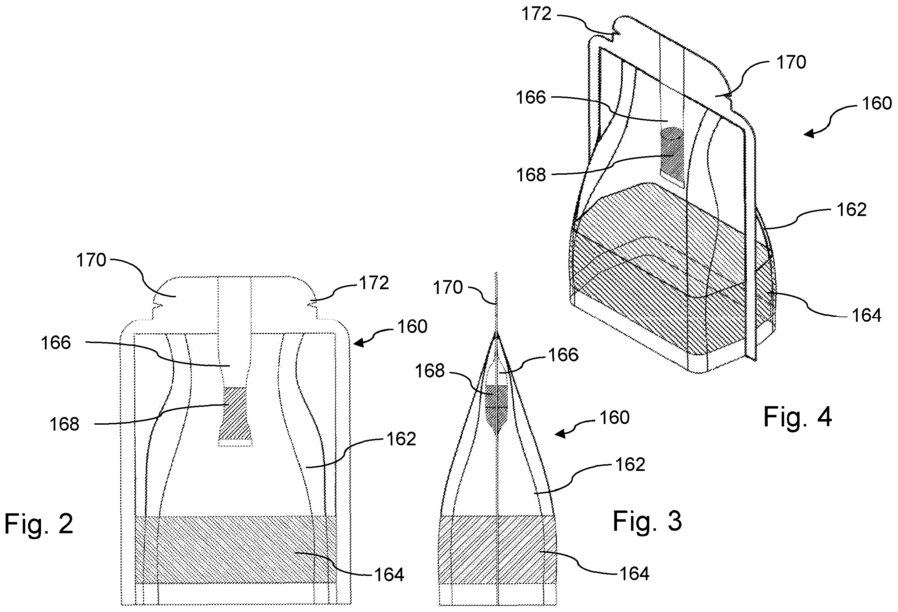

Figures 2 to 7 illustrate a cleaning apparatus according to a preferred embodiment of the invention. The cleaning apparatus may, in particular, provide a pre-clean apparatus 160 that can be used to provide an initial cleaning of an endoscope 2.

The apparatus 160 comprises a bag 162 including a main body portion containing a volume of a first liquid, which in this example is sterile water 164. The apparatus further comprises a pouch 166 containing a volume of a second liquid, which in this example is a concentrated detergent 168. The bag 162 includes a neck portion 170 that, when open, provides access to an internal volume of the main body portion. The neck portion is preferably of suitable dimensions to allow a tip or insertion portion of an endoscope 2 to be inserted through the neck 170 and into the main body portion of the bag 162.

In this embodiment the neck 170 of the bag 162 is closed at the top with a heat welded seal. The neck 170 may have two V-groove regions 172 on either side, proximate the top of the neck 170, which will allow a user to tear the top off the neck 170 to open the bag 162. It will be understood that the V-groove regions may provide a guide to a user as to where to tear the neck portion and may provide a point of weakness to assist a user in tearing the neck portion.

The reservoir of this embodiment in is the form of a narrow tubular bag or pouch 166, which is initially separate from the bag. This tubular pouch 166 preferably has a diameter small enough to sit inside the neck 170 of the larger bag 162. During manufacture of the cleaning apparatus, the top of the smaller tubular pouch 166 (reservoir) is preferably heat sealed at its top concurrently with a top of the neck 170 of the larger bag 162. Heat sealing may therefore be used to seal both the reservoir and the bag, and may also attach the reservoir to the bag. In this embodiment the reservoir pouch is attached to the bag so that the reservoir is suspended from a top edge of the neck portion of the bag and hangs down or extends into the main body portion of the bag. In this way, the concentrated detergent 168 is contained within the reservoir 166 in the neck 170 of the larger bag 162 isolated from the sterile water 164 within the main internal volume of the bag 162.

The heat seal is preferably disposed above a line across which a user is guided to tear or cut open the bag to gain access to the internal volume of the bag. Accordingly, when the user tears open the neck 170 of the larger bag 162, the smaller reservoir 166 (now detached from the bag) falls into the main body portion of the larger bag 162. The second liquid (concentrated detergent) 168 is then mixed with the first liquid (sterile water) 164. As such, the lower concentration detergent solution is only formed when a user is ready to use the cleaning apparatus.

This configuration of the reservoir and bag therefore provides a low cost apparatus 160, not requiring additional mouldings.

**In** preferred embodiments, to allow the cleaning apparatus to be used with medical equipment such as an endoscope, the neck 170 of the bag 162 preferably has a large enough diameter to allow a tip portion or insertion portion of an endoscope 2 to be easily inserted into the main body portion of the bag.

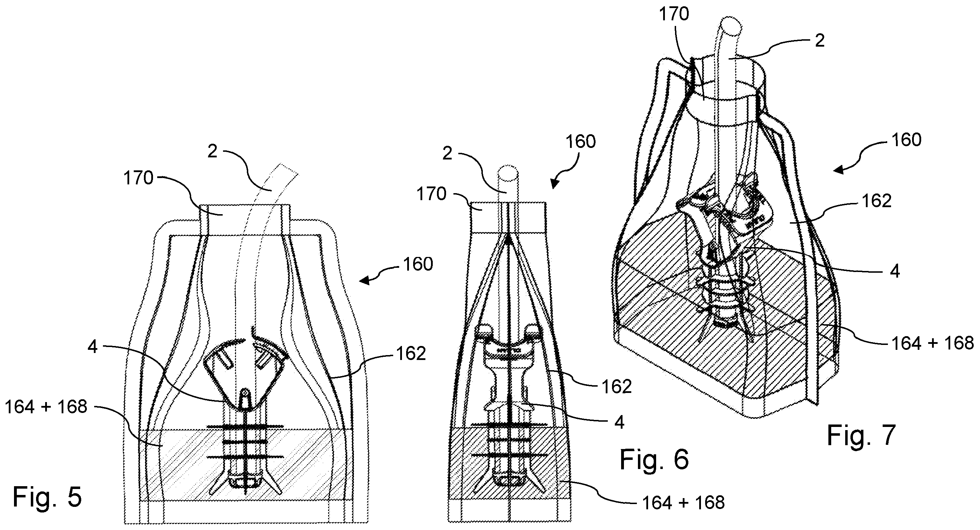

**In** some cases an endoscope may be fitted with a tip protector 4 (such as that disclosed in EP3206555), as illustrated in Figure 5 to 7. Accordingly, it may be preferable for the neck of the bag to have a diameter large enough to allow the tip portion of the endoscope with the tip protector attached to be inserted into the bag 162 and removed from the bag 162 through the neck 170 without the tip protector 4 being pulled off the end of the endoscope 2.

In some applications, a cylindrical sponge is used to wipe detergent onto the endoscope, in particular onto external surfaces of the endoscope. In some examples the cylindrical sponge has a hole through the centre and a slit in one side and is designed to fit around the endoscope insertion tube. In use, a user slides the sponge along the endoscope insertion tube and agitates it back and forth to clean the endoscope. In these cases, the neck of the bag preferably has dimensions large enough to accommodate this sponge so that a user may use a detergent solution within the bag to clean external surfaces of the endoscope. Typically, this type of sponge may be 50mm in diameter and 100mm long.

In preferred embodiments, and in particular for use in flushing and cleaning endoscopes, the bag contains 500ml of sterile water and the reservoir contains 5ml of concentrated detergent.

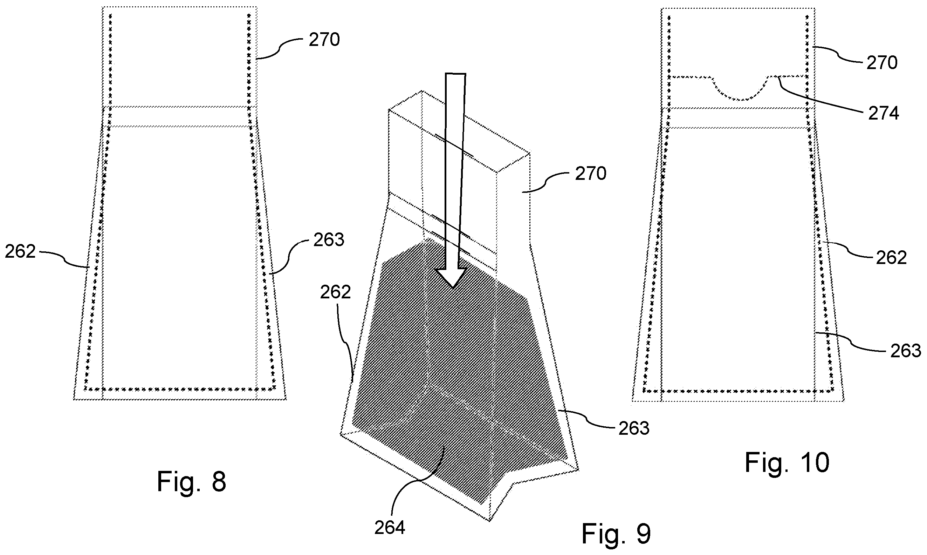

Figures 8 to 19 illustrate an alternative example of a pre-clean apparatus 260.

Figures 8 to 13 illustrate the features of the cleaning apparatus 260 with reference to method steps involved in manufacturing the cleaning apparatus 260. In this embodiment the cleaning apparatus comprises a bag 262 including a main body portion 263 and a neck portion 270 extending upwardly from the main body portion 263. Sides and a base of the bag are sealed (as indicated by dotted lines in Figure 8) to form an internal volume of the bag 262. Initially an upper end of the neck portion 270 is open to allow the bag 262 to be filled with a first volume of liquid, as illustrated in Figure 9. This first volume of liquid is preferably sterile or distilled water 264.

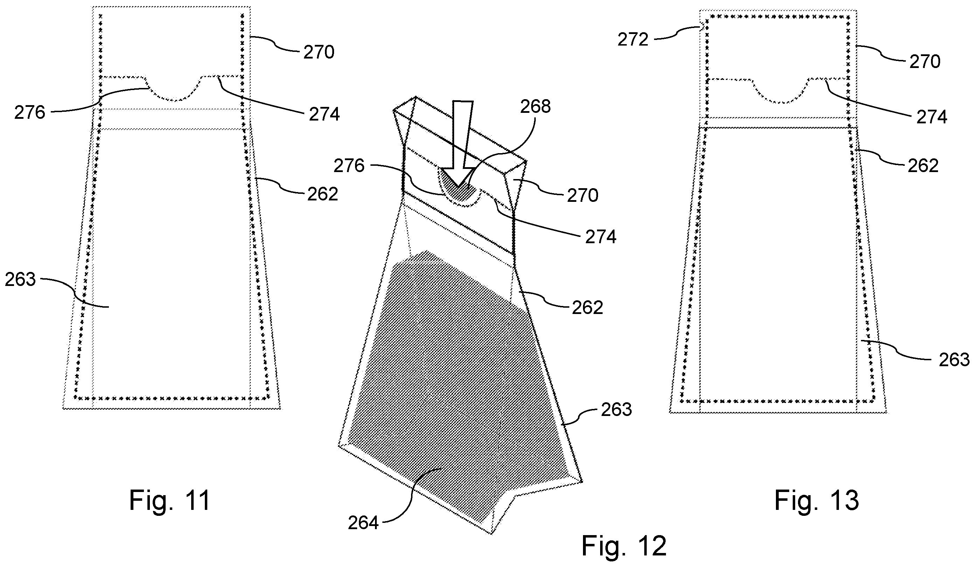

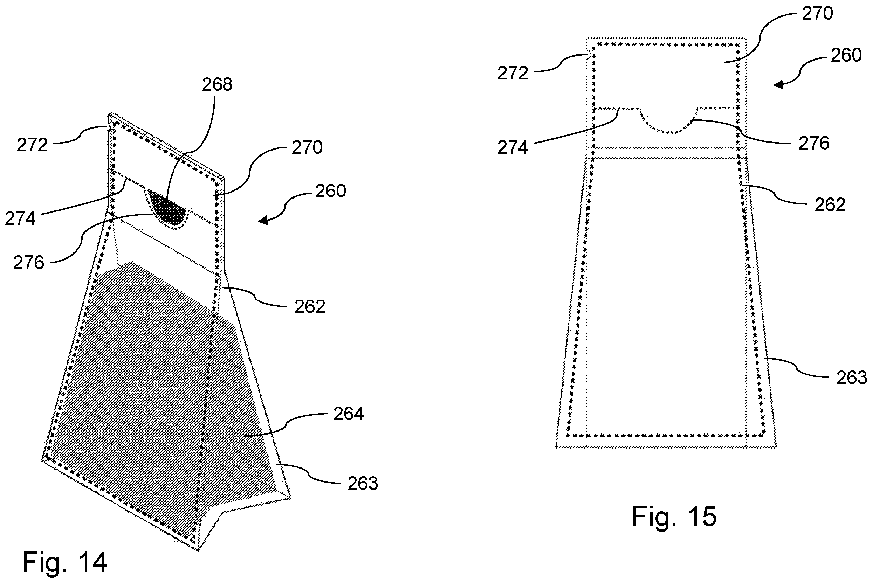

After the first liquid 264 has been poured into the bag 262, a shaped frangible seal 274 is welded across the neck portion 270, as shown in Figure 10. This frangible seal 274 seals the main body portion 263 of the bag 262 to contain the first liquid (sterile water) 264 within the bag 262. The shape of the frangible seal 274, as best shown in Figure 11 includes a recess or concavity 276 for receiving a volume of a second liquid, which may be concentrated detergent. To form the recess 276, in this embodiment the frangible seal 274 comprises a weld line that extends in a direction generally across the neck portion 270, with a central region of the weld line having a semi-circular shape that extends generally in a direction towards the base of the bag 262. In a next step, this recess 276 is filled with concentrated detergent 268, as shown in Figure 12. The upper end of the neck portion 270 is then sealed, as shown in Figure 13. The seal closing the upper end of the neck portion 270 retains the second liquid (concentrated detergent) in a region of the neck portion between the upper seal and the frangible seal 274. The arrangement of the seals therefore means that the concentrated detergent (second liquid) 268 is held in the neck portion 270 of the bag 262 separated from the sterile water (first liquid) 264 in the main body 263 of the bag 262, as shown in Figure 14.

A tear slot or cut-out 272 may be provided at one side of the neck portion 270 proximate its upper end. This provides a weakened area of weld to allow a user to subsequently tear open the neck portion 270 of the bag 262 at that point. It will be understood that the tear slot 272 is disposed below the upper seal but above the frangible seal.

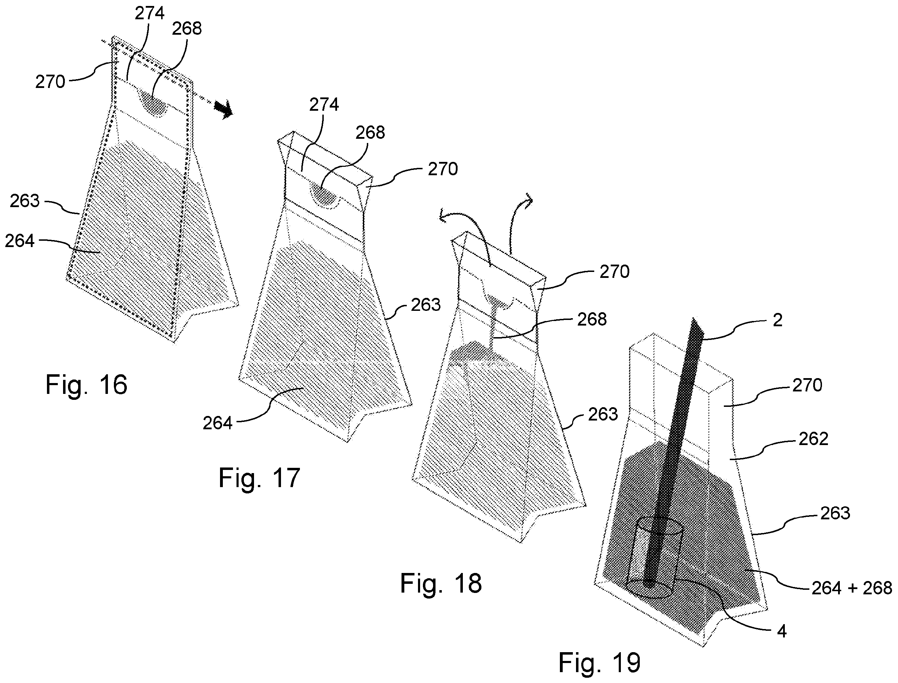

To use the pre-clean apparatus 260, a user first tears or cuts open the upper end of the neck portion 270, below the upper seal and above the frangible seal, as shown in Figure 16. Subsequently, by pulling on the sides of the neck portion 270 at its upper end, a user can break the frangible seal 274 without tearing the walls of the bag 262. This dispenses the concentrated detergent 268 into the sterile water 264 as shown in Figure 18. The detergent mixes with the water in the main body 263 of the bag 262.

An endoscope 2 may then be inserted into the main body portion 263 of the bag 262 through the neck portion 270, as illustrated in Figure 19. A tip section of the endoscope 2 may be cleaned with the detergent solution within the bag 262, with optional use of a suitable sponge as described above. The detergent solution may also be drawn up through the channels of the endoscope to perform a cleaning flush of the channels of the endoscope, in particular a suction/biopsy channel of the endoscope.

In preferred embodiments, the neck portion of the bag includes a retainer for holding the tip of the instrument or piece of equipment within the volume of liquid in the bag during use. The retainer may be in the form of an elastic or sprung clip that grips a part of the instrument or equipment that extends through the neck portion of the bag. The retainer may, for example, be in the form of an elastically deformable ring engaged with the neck portion of the bag. The deformable ring may be biased into a position in which a narrow opening is provided in the neck portion and the deformable ring must be pressed or stretched to increase a dimension of the opening to allow the instrument or piece of equipment to be inserted through the neck portion. With the force on the deformable ring released the ring tries to return to its original position and grips a part of the instrument or piece of equipment.

Alternatively the retainer may comprise a tie, for example a cable tie or similar. In use the tie may be used to tighten a part of the neck portion around a part of the instrument or piece of equipment. The retainer may comprise a first region of the neck portion that may be adhered to a second region of the neck portion. One or both of the first and second regions may include a layer of adhesive. In use the first and second regions may be adhered to each other to effectively wrap the neck portion around a part of the instrument or piece of equipment. The layer of adhesive may be exposed when a user opens the top of the bag to use the cleaning apparatus.

The retainer maybe integral with or separate from the neck portion of the bag.

As described above, in some applications a cylindrical sponge may be used to wipe detergent onto the endoscope. The cylindrical sponge may have a hole or bore through the centre and a slit in one side such that it fits around the endoscope insertion tube. In these cases, the retainer may be configured to grip or clamp the sponge around the endoscope to retain the tip of the endoscope in the bag. This has the advantage that the sponge provides a layer of protection between the retainer and the insertion tube of the endoscope.

Although in the above embodiment the frangible seal was shaped to provide a recess, it will be appreciated that in other embodiments the frangible seal may extend straight across the neck of the bag, or the frangible seal may include a weld line having any suitable shape to form a sealed boundary of the reservoir to initially separate the first and second liquids.

In the examples illustrated above, the bag is shown as having a generally square or rectangular base. This allows the bag to more easily stand upright on a suitable surface during use. It will be appreciated, however, that the bag may be of any suitable shape. In some examples in which it is beneficial for the bag to be stood upright, the bag may be of any suitable shape and either a lower end of the bag may be placed into a receptacle or dish having a suitable shape, or a perforated plate or dish may be included in the internal volume of the bag. In examples in which a perforated plate or dish is provided, this plate or dish may initially be disposed in a central region of the internal volume of the bag and then a user may push the plate or dish towards a base region of the bag to form a flat base to aid in standing the bag on a surface.

Other modifications and variations not explicitly disclosed above may also be contemplated without departing from the scope of the invention as defined in the appended claims.

## Claims

1. A cleaning apparatus (260) for cleaning internal channels of an item of equipment, the apparatus comprising:
a bag (262) including a main body portion (263) and a neck portion (270) through which, in use, a part of said item of equipment is inserted to access to the main body portion (263) of the bag (262), the main body portion (263) providing an internal volume for holding a volume of sterile or distilled water, and the neck portion (270) including an upper seal at an end furthest from the main body portion (263);
a frangible seal (274) extending across the neck portion (270) of the bag (262) thereby forming a fluid boundary between the internal volume of the main body portion (263) and an upper region of the neck portion (270);
a reservoir (276) having an internal volume for holding a volume of detergent, the reservoir being disposed in the neck portion (270) of the bag (262) between the frangible seal (274) and the upper seal, such that, in use, when the neck portion (270) is opened to access the internal volume of the main body portion (263), the volume of detergent is concurrently released from the reservoir (276) to mix with the volume of sterile or distilled water in the main body portion (263); and
a retainer in the neck portion (270) of the bag (262) arranged, in use, to engage said item of equipment to hold a part of said item of equipment in said mixed volume of detergent and sterile or distilled water.

2. A cleaning apparatus according to Claim 1, in which the internal volume of the reservoir (276) is no more than 10% of the internal volume of the main body portion (263).

3. A cleaning apparatus according to Claim 1 or Claim 2, in which the internal volume of the reservoir (276) holds 5ml of liquid and the internal volume of the main body portion (263) holds 500ml of liquid.

4. A cleaning apparatus according to any preceding claim, in which the retainer comprises an elastic or sprung clip, an elastically deformable ring, a tie, or adhesive regions of the neck portion.

5. A cleaning apparatus according to any preceding claim, in which the frangible seal (274) includes a weld line having an arc-shaped portion forming a concavity for receiving the second liquid.

6. A cleaning apparatus according to any preceding claim, in which the upper seal is a heat welded seal.

7. A method of manufacturing a cleaning apparatus (260), the apparatus being according to any one of Claims 1 to 6, and the method comprising:
filling the main body portion (263) of the bag (262) with a first volume of the sterile or distilled water;
forming a frangible seal (274) across the neck portion (270) of the bag (262) to provide a fluid boundary between the main body portion (263) of the bag (262) and an upper region of the neck portion (270);
filling a part of the neck portion (270) with a second volume of the detergent; and
sealing a top of the neck portion (270) furthest from the main body portion (263), such that the detergent is in a sealed volume between the frangible seal (274) and the top of the neck portion (270).

8. A method of manufacturing a cleaning apparatus according to Claim 7, in which the top of the neck portion (270) is sealed by heat welding.

9. A method of manufacturing a cleaning apparatus according to Claim 7 or Claim 8, in which the frangible seal (274) comprises a weld line that extends in a direction generally across the neck portion (270) with a central region of the weld line having a semi-circular shape that extends generally in a direction towards the main body portion (263) and defines a recess (276) of the frangible seal (274), and the method comprises filling the recess (276) with the detergent.

10. A method of using a cleaning apparatus, the cleaning apparatus being according to any one of Claims 1 to 6, and the method comprising:
breaking the upper seal or separating the upper seal from the bag (262);
breaking the frangible seal (274) to dispense the volume of detergent into the volume of sterile or distilled water in the main body portion (263) of the bag (262); and
inserting a piece of equipment through the neck portion (270) of the bag (262) into the liquid in the main body portion (263) of the bag (262).

11. A method of using a cleaning apparatus according to Claim 10, in which the piece of equipment is an endoscope and the method comprises inserting a tip of the endoscope into the liquid in the main body portion (263) of the bag (262).

12. A method of using a cleaning apparatus according to Claim 10 or Claim 11, in which the neck portion (270) of the bag (262) comprises a retainer and the method comprises engaging the retainer with the item of equipment to retain a part of the item of equipment in the liquid in the main body portion (263) of the bag (262).

## Patentansprüche

1. Reinigungsvorrichtung (260) zum Reinigen innerer Kanäle eines Ausrüstungsgegenstands, wobei die Vorrichtung umfasst:
einen Beutel (262), der einen Hauptkörperabschnitt (263) und einen Halsabschnitt (270) aufweist, durch den bei Gebrauch ein Teil des Ausrüstungsgegenstands eingeführt wird, um Zugang zu dem Hauptkörperabschnitt (263) des Beutels (262) zu erhalten, wobei der Hauptkörperabschnitt (263) ein Innenvolumen zum Aufnehmen eines Volumens sterilen oder destillierten Wassers bereitstellt und der Halsabschnitt (270) an einem von dem Hauptkörperabschnitt (263) am weitesten entfernten Ende eine obere Versiegelung aufweist;
eine aufbrechbare Versiegelung (274), die sich quer über den Halsabschnitt (270) des Beutels (262) erstreckt und dadurch eine Fluidgrenze zwischen dem Innenvolumen des Hauptkörperabschnitts (263) und einem oberen Bereich des Halsabschnitts (270) bildet;
ein Reservoir (276) mit einem Innenvolumen zum Aufnehmen eines Volumens eines Reinigungsmittels, wobei das Reservoir in dem Halsabschnitt (270) des Beutels (262) zwischen der aufbrechbaren Versiegelung (274) und der oberen Versiegelung so angeordnet ist, dass im Gebrauchszustand, wenn der Halsabschnitt (270) geöffnet wird, um Zugang zu dem Innenvolumen des Hauptkörperabschnitts (263) zu erhalten, das Volumen des Reinigungsmittels gleichzeitig aus dem Reservoir (276) freigesetzt wird, so dass es sich mit dem Volumen sterilen oder destillierten Wassers in dem Hauptkörperabschnitt (263) vermischen kann; und
einen Halter im Halsabschnitt (270) des Beutels (262), der im Gebrauchszustand dazu eingerichtet ist, mit dem Ausrüstungsgegenstand in Eingriff zu treten, um einen Teil des Ausrüstungsgegenstands in dem gemischten Volumen aus Reinigungsmittel und sterilem oder destilliertem Wasser zu halten.

2. Reinigungsvorrichtung nach Anspruch 1,
bei welcher das Innenvolumen des Reservoirs (276) nicht mehr als 10% des Innenvolumens des Hauptkörperabschnitts (263) beträgt.

3. Reinigungsvorrichtung nach Anspruch 1 oder Anspruch 2, bei welcher das Innenvolumen des Reservoirs (276) 5 ml Flüssigkeit aufnimmt und das Innenvolumen des Hauptkörperabschnitts (263) 500 ml Flüssigkeit aufnimmt.

4. Reinigungsvorrichtung nach einem der vorigen Ansprüche, bei welcher der Halter einen elastischen oder federnden Clip, einen elastisch verformbaren Ring, ein Band oder Klebebereiche des Halsabschnitts umfasst.

5. Reinigungsvorrichtung nach einem der vorigen Ansprüche, bei welcher die aufbrechbare Versiegelung (274) eine Schweißnaht mit einem bogenförmigen Abschnitt aufweist, der eine Konkavität zum Aufnehmen der zweiten Flüssigkeit ausbildet.

6. Reinigungsvorrichtung nach einem der vorigen Ansprüche, bei welcher die obere Versiegelung eine heißverschweißte Versiegelung ist.

7. Verfahren zur Herstellung einer Reinigungsvorrichtung (260), bei welchem die Vorrichtung nach einem der Ansprüche 1 bis 6 ausgebildet ist und das Verfahren folgendes umfasst:
Befüllen des Hauptkörperabschnitts (263) des Beutels (262) mit einem ersten Volumen des sterilen oder destillierten Wassers;
Ausbilden einer aufbrechbaren Versiegelung (274) quer über den Halsabschnitt (270) des Beutels (262), um eine Fluidgrenze zwischen dem Hauptkörperabschnitt (263) des Beutels (262) und einem oberen Bereich des Halsabschnitts (270) bereitzustellen;
Befüllen eines Teils des Halsabschnitts (270) mit einem zweiten Volumen des Reinigungsmittels; und
Versiegeln einer Oberseite des Halsabschnitts (270), die am weitesten von dem Hauptkörperabschnitt (263) entfernt ist, derart, dass sich das Reinigungsmittel in einem versiegelten Volumen zwischen der aufbrechbaren Versiegelung (274) und der Oberseite des Halsabschnitts (270) befindet.

8. Verfahren zur Herstellung einer Reinigungsvorrichtung nach Anspruch 7,
bei welchem die Oberseite des Halsabschnitts (270) durch Heißverschweißen versiegelt wird.

9. Verfahren zur Herstellung einer Reinigungsvorrichtung nach Anspruch 7 oder Anspruch 8,
bei welchem die aufbrechbare Versiegelung (274) eine Schweißnaht umfasst, die sich in einer Richtung im Wesentlichen quer über den Halsabschnitt (270) erstreckt, wobei ein mittlerer Bereich der Schweißnaht eine halbkreisförmige Form aufweist, die sich im Wesentlichen in Richtung des Hauptkörperabschnitts (263) erstreckt und eine Ausnehmung (276) der aufbrechbaren Versiegelung (274) definiert, und wobei das Verfahren das Befüllen der Ausnehmung (276) mit dem Reinigungsmittel umfasst.

10. Verfahren zur Verwendung einer Reinigungsvorrichtung, wobei die Reinigungsvorrichtung nach einem der Ansprüche 1 bis 6 ausgebildet ist und das Verfahren folgendes umfasst:
Aufbrechen der oberen Versiegelung oder Trennen der oberen Versiegelung von dem Beutel (262);
Aufbrechen der aufbrechbaren Versiegelung (274), um das Volumen des Reinigungsmittels in das Volumen sterilen oder destillierten Wassers in dem Hauptkörperabschnitt (263) des Beutels (262) abzugeben; und
Einführen eines Ausrüstungsgegenstands durch den Halsabschnitt (270) des Beutels (262) in die Flüssigkeit in dem Hauptkörperabschnitt (263) des Beutels (262).

11. Verfahren zur Verwendung einer Reinigungsvorrichtung nach Anspruch **10,**
bei welchem der Ausrüstungsgegenstand ein Endoskop ist und das Verfahren das Einführen einer Spitze des Endoskops in die Flüssigkeit in dem Hauptkörperabschnitt (263) des Beutels (262) umfasst.

12. Verfahren zur Verwendung einer Reinigungsvorrichtung nach Anspruch 10 oder Anspruch 11,
bei welchem der Halsabschnitt (270) des Beutels (262) einen Halter umfasst und das Verfahren umfasst, den Halter mit dem Ausrüstungsgegenstand in Eingriff zu bringen, um einen Teil des Ausrüstungsgegenstands in der Flüssigkeit in dem Hauptkörperabschnitt (263) des Beutels (262) zurückzuhalten.

## Revendications

1. - Appareil de nettoyage (260) pour nettoyer des canaux internes d'un article d'équipement, l'appareil comprenant :
un sac (262) comprenant une partie corps principal (263) et une partie col (270) à travers laquelle, lors de l'utilisation, une partie dudit article d'équipement est introduite pour accéder à la partie corps principal (263) du sac (262), la partie corps principal (263) offrant un volume interne destiné à contenir un volume d'eau stérile ou distillée, et la partie col (270) comprenant un joint d'étanchéité supérieur à une extrémité la plus éloignée de la partie corps principal (263) ;
un joint d'étanchéité cassable (274) s'étendant à travers la partie col (270) du sac (262), formant ainsi une limite de fluide entre le volume interne de la partie corps principal (263) et une région supérieure de la partie col (270) ;
un réservoir (276) ayant un volume interne destiné à contenir un volume de détergent, le réservoir étant disposé dans la partie col (270) du sac (262) entre le joint d'étanchéité cassable (274) et le joint d'étanchéité supérieur de telle sorte que, lors de l'utilisation, lorsque la partie col (270) est ouverte pour accéder au volume interne de la partie corps principal (263), le volume de détergent est simultanément libéré du réservoir (276) pour se mélanger avec le volume d'eau stérile ou distillée dans la partie corps principal (263) ; et
un dispositif de retenue dans la partie col (270) du sac (262) conçu, lors de l'utilisation, pour s'engager avec ledit article d'équipement afin de maintenir une partie dudit article d'équipement dans ledit volume mélangé de détergent et d'eau stérile ou distillée.

2. - Appareil de nettoyage selon la revendication 1, dans lequel le volume interne du réservoir (276) ne dépasse pas 10 % du volume interne de la partie corps principal (263).

3. - Appareil de nettoyage selon la revendication 1 ou la revendication 2, dans lequel le volume interne du réservoir (276) contient 5 ml de liquide et le volume interne de la partie corps principal (263) contient 500 ml de liquide.

4. - Appareil de nettoyage selon l'une quelconque des revendications précédentes, dans lequel le dispositif de retenue comprend une pince élastique ou à ressort, un anneau déformable élastiquement, une attache ou des zones adhésives de la partie col.

5. - Appareil de nettoyage selon l'une quelconque des revendications précédentes, dans lequel le joint d'étanchéité cassable (274) comprend une ligne de soudure ayant une partie en forme d'arc formant une concavité destinée à recevoir le second liquide.

6. - Appareil de nettoyage selon l'une quelconque des revendications précédentes, dans lequel le joint d'étanchéité supérieur est un joint d'étanchéité thermosoudé.

7. - Procédé de fabrication d'un appareil de nettoyage (260), l'appareil étant selon l'une quelconque des revendications 1 à 6, et le procédé comprenant :
remplir la partie corps principal (263) du sac (262) avec un premier volume d'eau stérile ou distillée ;
former un joint d'étanchéité cassable (274) à travers la partie col (270) du sac (262) afin de fournir une limite de fluide entre la partie corps principal (263) du sac (262) et une région supérieure de la partie col (270) ;
remplir une partie de la partie col (270) avec un second volume de détergent ; et
sceller de manière étanche une partie supérieure de la partie col (270) la plus éloignée de la partie corps principal (263), de telle sorte que le détergent se trouve dans un volume scellé de manière étanche entre le joint d'étanchéité cassable (274) et la partie supérieure de la partie col (270).

8. - Procédé de fabrication d'un appareil de nettoyage selon la revendication 7, dans lequel la partie supérieure de la partie col (270) est scellée de manière étanche par thermosoudage.

9. - Procédé de fabrication d'un appareil de nettoyage selon la revendication 7 ou la revendication 8, dans lequel le joint d'étanchéité cassable (274) comprend une ligne de soudure qui s'étend dans une direction de manière générale à travers la partie col (270), une région centrale de la ligne de soudure ayant une forme semi-circulaire qui s'étend de manière générale dans une direction vers la partie corps principal (263) et définit un évidement (276) du joint d'étanchéité cassable (274), et le procédé comprend le remplissage de l'évidement (276) avec le détergent.

10. - Procédé d'utilisation d'un appareil de nettoyage, l'appareil de nettoyage étant selon l'une quelconque des revendications 1 à 6, et le procédé comprenant :
rompre le joint d'étanchéité supérieur ou séparer le joint d'étanchéité supérieur du sac (262) ;
rompre le joint d'étanchéité cassable (274) pour distribuer le volume de détergent dans le volume d'eau stérile ou distillée dans la partie corps principal (263) du sac (262) ; et
introduire un élément d'équipement à travers la partie col (270) du sac (262) dans le liquide se trouvant dans la partie corps principal (263) du sac (262).

11. - Procédé d'utilisation d'un appareil de nettoyage selon la revendication 10, dans lequel l'élément d'équipement est un endoscope et le procédé comprend l'introduction d'une pointe de l'endoscope dans le liquide se trouvant dans la partie corps principal (263) du sac (262).

12. - Procédé d'utilisation d'un appareil de nettoyage selon la revendication 10 ou la revendication 11, dans lequel la partie col (270) du sac (262) comprend un dispositif de retenue et le procédé comprend l'engagement du dispositif de retenue avec l'élément d'équipement afin de retenir une partie de l'élément d'équipement dans le liquide se trouvant dans la partie corps principal (263) du sac (262).
